Europäisches Patentamt

(19) European Patent Office

Office européen des brevets

(11) Numéro de publication: **0 014 602**
**B1**

(12) **FASCICULE DE BREVET EUROPEEN**

(45) Date de publication du fascicule du brevet: **24.03.82**

(51) Int. Cl.³: **C 07 C 79/37**

(21) Numéro de dépôt: **80400004.0**

(22) Date de dépôt: **03.01.80**

(54) Nouveau procédé de préparation de mononitro-tétrahydro-1,2,3,4 anthraquinones.

(30) Priorité: **19.01.79 FR 7901325**

(43) Date de publication de la demande:
**20.08.80 Bulletin 80/17**

(45) Mention de la délivrance du brevet:
**24.03.82 Bulletin 82/12**

(84) Etats contractants désignés:
**BE CH DE FR GB IT NL**

(56) Documents cités:
**FR - A - 658 972**
**FR - A - 1 224 885**

(73) Titulaire: **P C U K PRODUITS CHIMIQUES UGINE KUHLMANN**
**Service Propriété Industrielle Tour Manhattan**
**F-92087 Paris La Defence 2 Cédex 21 (FR)**

(72) Inventeur: **Delavarenne, Serge Yvon**
**2, rue des Alouettes**
**F-69340 Francheville Le Haut (FR)**
Inventeur: **Dubreux, Bernard**
**29, avenue du Chater Bâtiment A 3**
**F-69340 Francheville Le Bas (FR)**
Inventeur: **Tellier, Pierre**
**106, avenue de la Libération**
**F-69110 Sainte Foy Les Lyon (FR)**

(74) Mandataire: **Houssin, Jean**
**P.C.U.K. - Produits Chimiques Ugine Kuhlmann**
**Service Propriété Industrielle Tour Manhattan - Cedex 21**
**F-92087 Paris La Defense 2 (FR)**

Courier Press, Leamington Spa, England.

# 0 014 602

Nouveau procédé de préparation de mononitro-tétrahydro-1, 2, 3, 4 anthraquinones

La présente invention concerne un procédé de préparation de dérivés mononitrés de la tétrahydro-1,2,3,4 anthraquinone à partir de la tétrahydro-1,4,4a,9a anthraquinone. Il est caractérisé par le fait que l'on réalise deux étapes successives: un prétraitement et une nitration.

Il est connu d'après l'art antérieur que les dérivés mononitrés de la tétrahydro-1,2,3,4 anthraquinone peuvent être obtenus par nitration soit du tétrahydro-1,2,3,4 anthracènediol-9, 10, soit de l'hexahydro-1,2,3,4,4a,9a anthraquinone, soit de la tétrahydro-1,2,3,4 anthraquinone. Ces derniers composés sont eux-mêmes obtenus à partir de la tétrahydro-1,4,4a,9a anthraquinone par une hydrogénation catalytique suivis éventuellement d'une réaction d'isomérisation ou d'une réaction d'oxydation. Or il a été trouvé dans les services de la demanderesse que l'on peut obtenir des mononitrotétrahydro-1,2,3,4 anthraquinones au départ de la tétrahydro-1,4,4a,9a anthraquinone à condition de soumettre préalablement cette dernière à un traitement thermique sous atmosphère inerte, en l'absence de réducteurs ou d'oxydants, en présence d'un catalyseur d'hydrogénation, ce qui conduit d'une part à l'anthraquinone, d'autre part à un mélange de tétrahydro-1,2,3,4 anthracènediol-9,10 et de tétrahydro-1,2,3,4 anthraquinone, dont la nitration conduit sélectivement aux mononitro 5- et 6- tétrahydro-1,2,3,4 anthraquinones. L'avantage d'un tel procédé par rapport à l'art antérieur réside essentiellement dans le fait que l'on évite une réaction d'hydrogénation catalytique at éventuellement une réaction d'oxydation et par conséquent le plus souvent la mise en oeuvre de technologies complexes inhérentes aux réactions effectuées sous pression.

Pour réaliser le procédé selon l'invention, on chauffe sous atmosphère inerte la tétrahydro-1,4,4a,9a anthraquinone, éventuellement dans un solvant, en présence d'un catalyseur d'hydrogénation. L'anthraquinone, le catalyseur et le mélange de tétrahydro-1,2,3,4 anthraquinone et de tétrahydro-1,2,3,4 anthracènediol-9,10 sont séparés par les techniques usuelles de la synthèse organique telles que filtration, évaporation, cristallisation... et on effectue ensuite la réaction de nitration sur le mélange de tétrahydro-1,2,3,4 anthraquinone et de tétrahydro-1,2,3,4 anthracènediol-9,10.

Pour la mise en oeuvre du prétraitement, parmi les solvants utilisables on choisit préférentiellement les alcools aliphatiques ou aromatiques monofonctionnels comprenant de 1 à 10 atomes de carbone. Parmi les alcools utilisables selon l'invention, on peut mentionner, par exemple, le méthanol, l'éthanol, le propanol et l'isopropanol, les butanols primaire et secondaire, le tertiobutanol, les alcools amyliques, les octanols, l'alcool benzylique, etc...

Les catalyseurs utilisables selon l'invention sont les catalyseurs couramment mis en oeuvre dans les réactions d'hydrogénation catalytique, par exemple, ceux à base de métaux précieux comme le palladium ou le platine ou ceux à base de nickel comme le nickel de Raney.

Pour le mise en oeuvre du procédé, on peut utiliser des solutions de tétrahydro-1,4,4a,9a anthraquinone à des concentrations pouvant varier entre 0,5 % en poids et la saturation à la température de la réaction. Cette dernière peut être comprise entre 20°C at 160°C, préférentiellement entre 70°C et 120°C. La quantité de catalyseur peut être comprise entre 0,01 at 20 % en poids par rapport à la quantité de précurseur engagé.

Pour effectuer la nitration du mélange de tétrahydro-1,2,3,4 anthraquinone et de tétrahydro-1,2,3,4 anthracènediol-9,10, on peut utiliser un mélange d'acide sulfurique et d'acide nitrique ou opérer dans l'acide nitrique pur. Il est également possible d'opérer en présence d'un solvant inerte dans les conditions de la réaction. Lorsqu'on opère avec de l'acide sulfurique et de l'acide nitrique, la concentration de l'acide sulfurique utilisée est supérieure à 70 %, de préférence supérieure à 90 %. On peut utiliser aussi de l'oléum à la place de l'acide sulfurique. La concentration de l'acide nitrique dépend alors de celle de l'acide sulfurique, mais elle est de préférence supérieure à 70 %. Lorsqu'on opère avec l'acide nitrique seul, la concentration de ce dernier est d'au moins 90% et de préférence d'au moins 98%. La nitration est effectuée avec une proportion molaire d'acide nitrique par rapport au mélange de tétrahydro-1,2,3,4 anthracènediol-9,10 et de tétrahydro-1,2,3,4 anthraquinone au moins égale à 1 ; préférentiellement elle est comprise entre 1 et 10. La réaction est effectuée à une température comprise entre 0 et 50°C, préférentiellement entre 0 et 30°C.

Les exemples suivants illustrent de façon non limitative la présente invention. Les exemples 1 à 3 concernent le prétraitement de la tétrahydro-1,4,4a,9a anthraquinone; les exemples 4 à 6 décrivent la nitration du mélange de tétrahydro-1,2,3,4 anthraquinone et de tétrahydro-1,2,3,4 anthracènediol-9,10.

### Exemple 1

Dans 100 ml d'alcool amylique on dissout 5 g de tétrahydro-1,4,4a,9a anthraquinone. A cette solution on ajoute 1 g de palladium à 5 % sur charbon. L'ensemble est porté à 120°C pendant 5 heures dans un réacteur agité et maintenu sous une atmosphère d'azote. Après refroidissement, on sépare un précipité que l'on lave avec 100 ml d'acétone. A partir du précipité, on récupère après extraction 1 g d'anthraquinone. Après concentration des phases organiques, on obtient d'autre part 3,8 g d'un mélange de tétrahydro-1,2,3,4 anthracènediol-9,10 et de tétrahydro-1,2,3,4 anthraquinone (Mélange A).

2

## 0 014 602

### Exemple 2

On opère comme dans l'exemple 1, mais à 70°C au lieu de 120°C. Au départ de 15 g de tétrahydro-1,4,4a,9a anthraquinone, on obtient 4,5 g d'anthraquinone et 10 g d'un mélange de tétrahydro-1,2,3,4 anthracènediol-9,10 et de tétrahydro-1,2,3,4 anthraquinone (Mélange B).

### Exemple 3

Sous atmosphère d'azote on chauffe une heure à 130°C 20 g de tétrahydro-1,4,4a,9a anthraquinone et 2 g de palladium à 5 % sur charbon. Après refroidissement, le mélange solide est lavé par 250 ml d'acétone. Après un traitement analogue à celui des exemples précédents, on obtient 3,6 g d'anthraquinone et 16 g d'un mélange de tétrahydro-1,2,3,4 anthracènediol-9,10 et de tétrahydro-1,2,3,4 anthraquinone (mélange C).

### Exemples 4, 5 et 6.

Dans un réacteur agité maintenu à 5°C et contenant un mélange d'acide sulfurique à 96 % et d'acide nitrique 100 % dans une proportion pondérale de 5 à 1, on ajoute en l'espace de 30 minutes un poids de mélange A, B ou C de tétrahydro-1,2,3,4 anthraquinone et de tétrahydro-1,2,3,4 anthracènediol-9,10 égal au 1/5 du poids de la solution sulfonitrique. On maintient la température de cette suspension entre 15 et 18°C pendant 3 heures. On filtre le précipité que l'on lave ensuite à l'eau et sèche. Le filtrat, dilué dans l'eau, donne un second précipité que l'on sépare, lave à l'eau et sèche. Le premier précipité est de la nitro-5 tétrahydro-1,2,3,4 anthraquinone pratiquement pure ; le second précipité est un mélange de nitro-5 et de nitro-6 tétrahydro-1,2,3,4 anthraquinone.

On obtient les résultats suivants :

| Exemple | Mélange/poids | Nitro-5-tétrahydro-1,2,3,4-anthraquinone | Mélange de nitro-5 et de nitro-6-tétrahydro-1,2,3,4 anthraquinone |
|---------|---------------|------------------------------------------|-------------------------------------------------------------------|
| 4 | A /3,8 g | 2,9 g Fusion 184°C | 1,1 g |
| 5 | B /10 g | 7,8 g Fusion 185°C | 3,0 g |
| 6 | C /16 g | 12,3 g Fusion 184°C | 4,8 g |

### Revendications

1. Procédé de préparation de dérivés mononitrés de la tétrahydro-1,2,3,4 anthraquinone à partir de la tétrahydro-1,4,4a,9a anthraquinone caractérisé par le fait que l'on effectue un prétraitement thermique de la tétrahydro-1,4,4a,9a anthraquinone en présence d'un catalyseur d'hydrogénation sous atmosphère inerte et en l'absence d'oxydants ou de réducteurs, sépare l'anthraquinone sous-produite ainsi que le catalyseur et soumet le mélange de tétrahydro-1,2,3,4 anthracènediol-9,10 et de tétrahydro-1,2,3,4 anthraquinone obtenu à une réaction de nitration.

2. Procédé selon la revendication 1 caractérisé par le fait que le prétraitement de la tétrahydro-1,4,4a,9a anthraquinone est effectué en présence d'un catalyseur d'hydrogénation à base d'un métal précieux tel que palladium ou platine ou à base de nickel.

3. Procédé selon la revendication 2 caractérisé par le fait que le catalyseur d'hydrogénation utilisé est le palladium.

4. Procédé selon chacune des revendications 2 et 3 caractérisé par le fait que la concentration en poids du catalyseur d'hydrogénation est comprise entre 0,01 % et 20 % du poids de la tétrahydro-1,4,4a,9a anthraquinone mise an oeuvre.

5. Procédé selon chacune des revendications 1 à 4 caractérisé par le fait que le prétraitement de la tétrahydro-1,4,4a,9a anthraquinone est effectué à une température comprise entre 20°C et 160°C, de préférence entre 70°C at 120°C.

6. Procédé selon chacune des revendications 1 à 5 caractérisé par le fait que le prétraitement de la tétrahydro-1,4,4a,9a anthraquinone est effectué en présence d'un solvant.

7. Procédé selon la revendication 6 caractérisé par le fait que le solvant utilisé est un alcool aliphatique ou aromatique monofonctionnel ayant de 1 à 10 atomes de carbonne.

8. Procédé selon chacune des revendications 1 à 7 caractérisé par le fait que la concentration en poids de tétrahydro-1,4,4a,9a anthraquinone mise en oeuvre lors du prétraitement de cette dernière dans un solvant approprié est comprise entre 0,5% et la saturation dans ce solvant à la température de réaction.

3

9. Procédé selon la revendication 1 caractérisé par le fait que la nitration du mélange de tétrahydro-1,2,3,4 anthracènediol-9,10 et de tétrahydro-1,2,3,4 anthraquinone est effectuée dans l'acide nitrique pur.

10. Procédé selon la revendication 1 caractérisé par le fait que la nitration du mélange de tétrahydro-1,2,3,4 anthracènediol-9,10 et de tétrahydro-1,2,3,4 anthraquinone est effectuée dans un mélange d'acide nitrique et d'acide sulfurique.

11. Procédé selon chacune des revendications 1, 9 et 10 caractérisé par le fait que l'on sépare sélectivement de la suspension obtenue la nitro-5 tétrahydro-1,2,3,4 anthraquinone.

**Patentansprüche**

1. Verfahren zur Herstellung von Mononitroderivaten von 1,2,3,4-Tetrahydroanthrachinon ausgehend von 1,4,4a,9a-Tetrahydroanthrachinon, dadurch gekennzeichnet, daß man 1,4,4a,9a-Tetrahydroanthrachinon in Gegenwart eines Hydrierkatalysators in inerter Atmosphäre und in Abwesenheit von Oxidationsmitteln oder Reduktionsmitteln einer thermischen Vorbehandlung unterwirft, das in dieser Weise als Nebenprodukt gebildete Anthrachinon sowie den Katalysator abtrennt und die erhaltene Mischung aus 1,2,3,4-Tetrahydroanthracen-9,10-diol und 1,2,3,4-Tetrahydroanthrachinon einer Nitrierungsreaktion unterwirft.

2. Verfahren nach Anspruch 1, dadurch gekennzeichnet, daß man die Vorbehandlung von 1,4,4a,9a-Tetrahydroanthrachinon in Gegenwart eines Hydrierkatalysators auf der Grundlage eines Edelmetalls, wie Palladium oder Platin oder auf der Grundlage von Nickel durchführt.

3. Verfahren nach Anspruch 2, dadurch gekennzeichnet, daß man als Hydrierkatalysator Palladium verwendet.

4. Verfahren nach einem der Ansprüche 2 und 3, dadurch gekennzeichnet, daß die auf das Gewicht bezogene Konzentration des Hydrierkatalysators zwischen 0,01 und 20 % des Gewichts des eingesetzten 1,4,4a,9a-Tetrahydroanthrachinons liegt.

5. Verfahren nach einem der Ansprüche 1 bis 4, dadurch gekennzeichnet, daß man die Vorbehandlung des 1,4,4a,9a-Tetrahydroanthrachinons bei einer Temperatur zwischen 20 und 160°C, vorzugsweise zwischen 70 und 120°C durchführt.

6. Verfahren nach einem der Ansprüche 1 bis 5, dadurch gekennzeichnet, daß man die Vorbehandlung des 1,4,4a,9a-Tetrahydroanthrachinons in Gegenwart eines Lösungsmittels durchführt.

7. Verfahren nach Anspruch 6, dadurch gekennzeichnet, daß man als Lösungsmittel einen aliphatischen oder aromatischen monofunktionellen Alkohol mit 1 bis 10 Kohlenstoffatomen verwendet.

8. Verfahren nach einem der Ansprüche 1 bis 7, dadurch gekennzeichnet, daß die auf das Gewicht bezogene Konzentration des der Vorbehandlung unterworfenen 1,4,4a,9a-Tetrahydroanthrachinons in einem geeigneten Lösungsmittel zwischen 0,5 % und der Sättigungskonzentration bei der Reaktionstemperatur in dem Lösungsmittel liegt.

9. Verfahren nach Anspruch 1, dadurch gekennzeichnet, daß man die Nitrierung der Mischung aus 1,2,3,4-Tetrahydroanthracen-9,10-diol und 1,2,3,4-Tetrahydroanthrachinon in reiner Salpetersäure bewirkt.

10. Verfahren nach Anspruch 1, dadurch gekennzeichnet, daß man die Nitrierung der Mischung aus 1,2,3,4-Tetrahydroanthracen-9,10-diol und 1,2,3,4-Tetrahydroanthrachinon in einer Mischung aus Salpetersäure und Schwefelsäure durchführt.

11. Verfahren nach einem der Ansprüche 1, 9 und 10, dadurch gekennzeichnet, daß man 5-Nitro-1,2,3,4-tetrahydroanthrachinon selektiv aus der erhaltenen Suspension abtrennt.

**Claims**

1. Process for the preparation of mononitro derivatives of 1,2,3,4-tetrahydro-anthraquinone from 1,4,4a,9a-tetrahydroanthraquinone, characterised in that a thermal pretreatment of the 1,4,4a,9a-tetrahydro anthraquinone is effected in the presence of a hydrogenation catalyst in an inert atmosphere and in the absence of oxidising or reducing agents, the anthraquinone by-product and the catalyst are separated and the mixture of 1,2,3,4-tetrahydro-9,10-anthracenediol and 1,2,3,4-tetrahydro-anthraquinone obtained is subjected to a nitration reaction.

2. Process according to claim 1, in which the pretreatment of the 1,4,4a,9a-tetrahydro-anthraquinone is effected in the presence of a hydrogenation catalyst based on a precious metal such as palladium or platinum or based on nickel.

3. Process according to claim 2 in which the hydrogenation catalyst used is palladium.

4. Process according to each of the claims 2 and 3, in which the concentration by weight of the hydrogenation catalyst is between 0.01% and 20% of the weight of the 1,4,4a,9a-tetrahydro-anthraquinone used.

5. Process according to each of the claims 1 to 4 in which the pretreatment of the 1,4,4a,9a-tetra-hydro-anthraquinone is effected at a temperature between 20°C and 160°C, preferably between 70°C and 120°C.

6. Process according to each of the claims 1 to 5, in which the pretreatment of the 1,4,4a,9a-tetrahydro-anthraquinone is effected in the presence of a solvent.

4

7. Process according to claim 6, in which the solvent used is an aliphatic or aromatic monofunctional alcohol having 1 to 10 carbon atoms.

8. Process according to each of the claims 1 to 7, in which the concentration by weight of 1,4,4a,9a-tetrahydro-anthraquinone used during the pretreatment of the latter in a suitable solvent is between 0.5% and the saturation in this solvent at the reaction temperature.

9. Process according to claim 1, in which the nitration of the mixture of 1,2,3,4-tetrahydro-9,10-anthracenediol and 1,2,3,4-tetrahydro-anthraquinone is effected in pure nitric acid.

10. Process according to claim 1, in which the nitration of the mixture of 1,2,3,4-tetrahydro-9,10-anthracenediol and 1,2,3,4-tetrahydro-anthraquinone is effected in a mixture of nitric and sulphuric acids.

11. Process according to each of the claims 1, 9 and 10, in which the 5-nitro-1,2,3,4-tetrahydro-anthraquinone is selectively separated from the suspension obtained.